# EUROPEAN PATENT APPLICATION

(11) **EP 4 671 357 A1**
(43) Date of publication of application: **31.12.2025**
(21) Application number: 24815821.4
(22) Date of filing: 27.05.2024
(51) Int. Cl.: C12M 3/06, C12M 1/12, C12M 1/00

(54) **MICROFLUIDIC DEVICE FOR FORMING THREE-DIMENSIONAL TISSUE BARRIER**

(30) Priority: 02.06.2023 KR 20230071505
(71) Applicant: Mepsgen Co., Ltd., Seoul 05836 (KR)
(72) Inventor: RHO, Hoon Suk, Seoul 06344 (KR); KIM, Yong Tae, Seoul 05855 (KR)
(74) Representative: Hauck Patent- und Rechtsanwälte PartmbB
(86) International application number: PCT/KR2024/007143
(87) International publication number: WO 2024/248445

(57) **Abstract**

The present invention relates to a microfluidic device for mimicking the structure and function of an in vivo tissue barrier. Specifically, the present invention relates to a microfluidic device which replaces an animal model by mimicking the structure and function of a 2D-3D connective tissue barrier, a 3D tissue barrier, and a 3D-3D tissue barrier, and thus may be used as a model for new drug development and toxicity assessment, a method for culturing cells in the microfluidic device, and a method for mimicking an organ or a tissue using the microfluidic device.

## Description

### [Technical Field]

The present invention relates to a microfluidic device which serves as an alternative to animal models by mimicking the structure and function of a 2D-3D connective tissue barrier, a 3D tissue barrier, and a 3D-3D tissue barrier, and thus can be used as a model for new drug development and toxicity assessment, a method for culturing cells in the microfluidic device, and a method for mimicking an organ or a tissue using the microfluidic device.

In addition, the present invention relates to a microfluidic device that addresses the shortcomings of existing microfluidic devices, thereby enabling easy evaluation of the morphology and function of cultured cells and tissue barriers.

### [Background Art]

To address the ethical concerns and cross-species differences associated with using experimental animal models to test drug efficacy and side effects, an organ-on-a-chip (OAC) has been developed, which integrates organ tissues on a chip. The organ-on-a-chip is based on a technology for culturing cells that constitute specific organs on a small chip, thereby mimicking the morphology and physiological characteristics of the organ and achieving desired functions. This allows for detailed studies of cell behavior and the mechanisms of physicochemical reactions in the microenvironment of specific organs, and may serve as a model for new drug development and toxicity assessment.

In the development of the organ-on-a-chip, existing two-dimensional (2D) cell culture models utilize petri dishes to culture cells in a two-dimensional manner. This approach fails to account for the tissue-specific differentiated functions of many cell types and fails to accurately predict in vivo tissue function and drug activity.

In particular, for brain tissue cells including neurons and astrocytes, which are in contact in a three-dimensional (3D) manner in vivo, the limitations of 2D cell culture models have led to a growing need for 3D cell culture models that accurately mimic the spatial structure and biochemical complexity of living tissue.

3D cell culture models closely mimic in vivo conditions, enabling directional growth and complex cell-to-cell connections in an in vitro experiment. Compared to 2D cell culture models, 3D cell culture models are useful for studying molecular-based tissue function, capturing signaling pathways and drug responsiveness in various disease states.

Meanwhile, advancements in microfluidics, coupled with advances in semiconductor processing technology, have enabled the mass production of microscale structures with high precision. In particular, photolithography and soft lithography technologies have made it possible to easily produce microchannel structures through stamping using semi-permanent molds. Through this, attempts have been made to mimic microstructures such as blood vessels or cell layers of organs of the human body in vitro.

Previous studies have utilized photolithography or soft lithography to fabricate chips composed of polydimethylsiloxane (PDMS) microchannels and porous membranes. Cells injected through inlets into microchannels connected between the inlets and outlets are then deposited in specific microchannel regions, allowing cell culture within the chip to replicate the cell layer structure of organs. Further, in multi-channel structures, the height difference between the central and side channels, and the surface tension differences caused by the curvature of the micropillars in structures where the central and side channels are divided into multiple micropillars, have been utilized to confine hydrogels to the central channel, forming a hydrogel structure for 3D cell culture.

However, organ chips utilizing microfluidic devices made of PDMS often suffer from drug or protein adsorption and loss on the PDMS surface, hindering their practical application in the pharmaceutical industry. Further, the method of injecting liquids into microchannels through the inlets requires careful attention to prevent bubble formation due to friction with the microchannel surface, necessitating a high level of operator skill. The ingress of air bubbles into the microchannels within the organ chips, exposing cells cultured within them to air, drastically reduces cell viability, resulting in an inability to form a tissue barrier.

In particular, the injection of high-viscosity hydrogels into the microchannels through the inlets requires greater expertise in controlling the injection rate. This makes multi-channel microfluidic devices, which utilize surface tension to confine the hydrogel to the central channel, difficult to apply in the pharmaceutical industry. Therefore, existing injection methods for multi-channel organ chips not only have low reproducibility in tissue barrier formation, but also present challenges in automating the entire organ mimicking process using mechanical and electronic control devices.

In addition, existing 3D organ chips using hydrogels can form 2D-3D tissue barriers because the hydrogel is confined in the central channel and hardened, preventing the addition of cells, proteins, and further hydrogels. This limitation hinders their ability to replicate the in vivo tissue structure and tissue-specific differentiation functions, including the 3D-3D tissue barrier, which are composed of a large number of cells and proteins.

Therefore, the inventors of the present invention have developed a microfluidic device to address the issues of multi-channel organ chips that inject cells, hydrogels, and solutions into the microchannels through the inlets. This device more closely mimics the structure and function of in vivo 3D-3D tissue barriers, thereby completing the present invention.

### (Prior Art Document)

### (Patent Document)

(Patent Document 1) Korean Patent No. 10-2313280 (Non-Patent Document)
(Non-Patent Document 1) Cho, H., et al. Three-dimensional blood-brain barrier model for in vitro studies of neurovascular pathology. Sci. Rep. 5, 15222 (2015).
(Non-Patent Document 2) Booth, R. & Kim, H. Characterization of a microfluidic in vitro model of the blood-brain barrier (µBBB). Lab Chip 12, 1784-1792 (2012).
(Non-Patent Document 3) Ahn, S. I., et al. Microengineered human blood-brain barrier platform for understanding nanoparticle transport mechanisms. Nature Communications 11.1, 175 (2020).

### [Summary of Invention]

### [Problems to be Solved by Invention]

An object of the present invention is to provide a microfluidic device that addresses ethical concerns and cross-species differences associated with the use of experimental animal models, and exhibits a structure and function more similar to in vivo tissue barriers than existing organ-on-a-chip devices by forming a 3D tissue barrier or a 3D-3D tissue barrier structure.

In addition, another object of the present invention is to provide a microfluidic device that suppresses the generation of bubbles within microchannels during solution injection, enables repeated injection of cells, proteins, and hydrogels, and utilizes a structure that enables material transfer through diffusion, thereby exhibiting a 3D-3D structure and function more similar to in vivo tissue barriers compared to existing organ-on-a-chip devices, and enables automation through mechanical and control devices.

### [Means for Solving Problems]

The present invention provides a microfluidic device including: an insert including a first central channel; a base including a first open chamber; and a porous membrane positioned between the insert and the base.

The present invention provides a method for culturing cells in a microfluidic device including: assembling the microfluidic device; inverting the microfluidic device, then injecting second cells into the first open chamber, and culturing the cells; injecting a hydrogel including third cells into the first open chamber, then culturing the cells; and inverting the microfluidic device again, then injecting first cells into the first central channel, and culturing the cells.

### [Advantageous Effects]

The microfluidic device of the present invention may be used for drug efficacy assessment by mimicking the major structures and substance transport phenomena of various tissue barriers in vivo, including the blood-brain barrier.

In addition, the microfluidic device of the present invention enables precise quantification of drug distribution around tissue barriers. In addition, since the insert is detachable from the base, cells included within the microfluidic device may be selectively isolated without damage and used for analysis. Further, the insert may be separated from the base, subjected to necessary treatments such as drug application, and then reassembled to observe cell responses.

Furthermore, the microfluidic device of the present invention utilizes a culture chamber having an open shape, enabling the injection of cells, hydrogels including cells, hydrogels including a 3D cell structure, hydrogels including biomaterials, and tissue samples collected from the human body. This allows for the realization of the 3D spatial structure and biochemical complexity of in vivo cells, thereby better mimicking the structure and function of tissue barriers.

### [Brief Description of Drawings]

FIGS. 1 and 2 are cross-sectional views of a microfluidic device.
FIG. 3 is a perspective view of the microfluidic device.
FIGS. 4 and 5 are exploded and assembled views of the microfluidic device.
FIG. 6 is a cross-sectional view of a microfluidic device including cells and a hydrogel.
FIGS. 7 and 8 are cross-sectional views illustrating the configuration and method for attaching and detaching the insert and base.
FIGS. 9 and 10 are perspective views of the insert.
FIG. 11 is a perspective view of the base.
FIG. 12 is a plan view of the base.
FIG. 13 is a bottom view of the base.
FIG. 14 is a perspective view of a cover.
FIG. 15 is a perspective view and a cross-sectional view illustrating the configuration and method for attaching and detaching the insert and base.
FIG. 16 is a plan view of a microfluidic device with an insert and a base attached thereto.
FIG. 17 is a cross-sectional view taken along line A-A' and an enlarged cross-sectional view of the microfluidic device with an insert and a base attached thereto shown in FIG. 16.
FIG. 18 is a front view of a microfluidic device with an insert and a base attached thereto.
FIG. 19 is a bottom view of a microfluidic device with an insert and a base attached thereto.
FIG. 20 is a perspective view of a microfluidic device including a cover.
FIG. 21 is a perspective view illustrating fluid flow within a microchannel included in the insert.
FIG. 22 is a schematic view illustrating a cell culture method within the microfluidic device.
FIG. 23 shows the morphology of human astrocytes cultured within a first open chamber after co-culturing human cerebral vascular pericytes, human astrocytes, and human cerebral microvascular endothelial cells derived from induced pluripotent stem cells in the microfluidic device.

### [Mode for Carrying out Invention]

Hereinafter, embodiments and examples of the present invention will be described in detail with reference to the accompanying drawings, so that those skilled in the art can readily practice the present invention. However, it should be understood that the present invention may be embodied in various forms and is not limited to the specific embodiments and examples described herein.

Throughout this specification, when a part is referred to as "including" a component, it should be understood that, unless explicitly stated otherwise, the term does not exclude the presence of other components but may further include additional components.

The present invention provides a microfluidic device including an insert including a first central channel; a base including a first open chamber; and a porous membrane positioned between the insert and the base.

As used herein, the term "microfluidic device" refers to a device including a microchannel configured to allow a fluid to flow on a substrate made of various materials, including plastic, glass, metal, or silicon, and which may include an organic polymer.

As used herein, the term "microchannel" refers to a channel having dimensions on the order of millimeters, micrometers, or nanometers, through which a fluid can flow, and is also referred to as a "mixing channel" in the present invention.

As used herein, the term "insert" refers to, but is not limited to, an object that can be inserted into a groove formed in another object.

In one embodiment, the insert may be detachable from the base, and the base may include a groove into which the insert can be inserted. Preferably, the insert includes a first latching head, and the base includes a second latching head, such that the first latching head and the second latching head engage each other, allowing the insert and the base to be detachably coupled in a snap-fit manner. In this case, to prevent leakage from the first central channel and the first open chamber, the porous membrane may act as a gasket by being subjected to strong pressure between the insert and the base.

The term "latching head" as used herein refers to a head that may be composed of an elastic material such as plastic, rubber, or silicone, and may be elastically deformed to achieve snap-fit attachment/detachment, but is not limited thereto.

The term "snap-fit" as used herein refers to a latching method in which a latching force is formed through the interaction of grooves that engage with each other, without the need for additional components or latching mechanisms.

In one embodiment, the first central channel may include an inlet and an outlet capable of inducing and controlling perfusion of a liquid. Methods for inducing and controlling the flow may include, but are not limited to, utilizing a hydrostatic pressure difference between the inlet and outlet, or connecting an external pump to the inlet and the outlet.

In one embodiment, the first open chamber may have a volume of 220 µL or less, and a surface area in contact with air of 44 mm² or less, to maintain the liquid within the chamber when the microfluidic device is inverted after liquid injection. In addition, the first open chamber may have a height (depth) of 5 mm or less, but is not limited thereto.

In one embodiment, the microfluidic device may be made of, but is not limited to, plastic, glass, metal, or silicon. Preferably, the microfluidic device is manufactured from plastic using injection molding technology.

In one embodiment, the first central channel may include tissue barrier cells.

The tissue barrier cells may include vascular endothelial cells, skin cells, cancer cells, glandular cells, muscle cells, and epithelial cells of the bronchial tubes, colon, small intestine, pancreas, or kidney. Preferably, they are vascular endothelial cells, and more preferably, induced pluripotent stem cell-derived human brain microvascular endothelial cells (iPSC-derived HBMECs) .

The term "cell" as used herein refers to biological cells, including plant cells, animal cells (e.g., mammalian cells), bacterial cells, and fungal cells.

The term "tissue barrier cell" as used herein refers to a cell that maintains the specific structure of a tissue and protects the tissue from external stimuli. In addition, it refers to a cell that utilizes strong binding between tissue barrier cells or with the extracellular matrix to selectively allow substances to pass through and maintain homeostasis in the concentration of substances within the tissue. The tissue barrier cells include epithelial tissue cells and vascular endothelial cells.

In one embodiment, the first open chamber may include internal tissue cells and hydrogels.

The internal tissue cells may include astrocytes, pericytes, neurons, neural stem cells, glial cells, cardiomyocytes, smooth muscle cells, intestinal epithelial cells, keratinocytes, dermal fibroblasts, podocytes, and glomerular endothelial cells. Preferably, the internal tissue cells are astrocytes or pericytes, and more preferably, human astrocytes (HA) or human brain vascular pericytes (HBVP).

As used herein, the term "internal tissue cells" refers to cells that constitute organs and tissues of the human body, including, but not limited to, epithelial tissue, muscle tissue, nervous tissue, or connective tissue, and includes all cells that constitute human tissue.

The hydrogel may be at least one selected from the group consisting of collagen, laminin, hyaluronic acid, minerals, fibrin, fibronectin, elastin, peptides, polyethylene glycol, and alginate. Preferably, the hydrogel is a collagen gel, a fibrin gel, a laminin gel, Matrigel, an animal-derived tumor basement membrane extract gel, a tissue decellularized extracellular matrix gel, a peptide gel, a polyethylene glycol gel, or an alginate gel, but is not limited thereto. More preferably, the hydrogel is a laminin gel or Matrigel.

The hydrogel may be injected from the outside of the microfluidic device into an upper portion of the first open chamber after the microfluidic device is inverted, and spatially confined within the first open chamber.

The term "hydrogel" as used herein refers to a material having a 3D polymer network structure that is cross-linked by cohesive forces such as covalent bonds, hydrogen bonds, van der Waals bonds, or physical bonds and may swell by containing a large amount of water inside an aqueous solution.

In one embodiment, the insert may be separated from the base, and one or more cells selected from the group consisting of cells included in the first central channel, cells included in the first open chamber, and cells attached to the porous membrane may be respectively isolated. Cell analysis, such as genetic analysis, may be performed using the isolated cells.

In one embodiment, the insert may be separated from the base, and the first central channel may be subjected to necessary treatment, such as drug application. The insert may then be reassembled with the base to observe cell responses.

The present invention provides a method for culturing cells in a microfluidic device, which includes: assembling a microfluidic device including an insert including a first central channel, a base including a first open chamber, and a porous membrane positioned between the insert and the base, wherein the insert is detachable from the base; inverting the microfluidic device, then injecting second cells into the first open chamber, and culturing the cells; injecting a hydrogel including third cells into the first open chamber, then culturing the cells; and inverting the microfluidic device again, then injecting first cells into the first central channel, and culturing the cells.

In one embodiment, the first central channel may include an inlet and an outlet capable of inducing and controlling perfusion of a liquid, the first open chamber may be open at an upper portion and in fluid communication with the outside, the hydrogel including the second cells and the third cells may be injected through the upper portion of the first open chamber, and the first cells may be injected through a first central channel inlet.

In one embodiment, the first cell may be selected from the group consisting of vascular endothelial cells, skin cells, cancer cells, glandular cells, muscle cells, and epithelial cells of the bronchial tube, colon, small intestine, pancreas, or kidney; the second cell may be selected from the group consisting of pericytes, glial cells, cardiomyocytes, smooth muscle cells, intestinal epithelial cells, keratinocytes, dermal fibroblasts, podocytes, and glomerular endothelial cells; and the third cell may be selected from the group consisting of astrocytes, neurons, and neural stem cells. Preferably, the first cell is a vascular endothelial cell, the second cell is a pericyte, and the third cell is an astrocyte. More preferably, the first cell is a human brain microvascular endothelial cell (HBMEC), the second cell is a human brain vascular pericyte (HBVP), and the third cell is a human astrocyte (HA). Most preferably, the first cell is an induced pluripotent stem cell-derived human brain microvascular endothelial cell (iPSC-derived HBMEC), the second cell is a human brain vascular pericyte (HBVP), and the third cell is a human astrocyte (HA).

In one embodiment, the present invention provides a method for culturing cells in a microfluidic device, which includes: assembling a microfluidic device including an insert including a first central channel, a base including a first open chamber, and a porous membrane positioned between the insert and the base, wherein the insert is detachable from the base; inverting the microfluidic device, then injecting 1 to 50 µl of human brain vascular pericytes (HBVPs) at a concentration of 10⁶ cells/ml into the first open chamber and culturing the cells; injecting a hydrogel including 1 to 50 µl of human astrocytes (HAs) at a concentration of 10³ cells/ml or more into the first open chamber and culturing the cells; inverting the microfluidic device again, and then injecting 1 to 50 µl of induced pluripotent stem cell-derived human brain microvascular endothelial cells (iPSC-derived HBMECs) at a concentration of 10⁵ cells/ml or more into the first central channel and culturing the cells.

The present invention provides a method for mimicking an organ or a tissue in a microfluidic device which includes: an insert including a first central channel; a base including a first open chamber; and a porous membrane positioned between the insert and the base, wherein the insert is detachable from the base.

The organ or tissue may include, but is not limited to, the blood-brain barrier (BBB), lungs, liver, heart, retina, large intestine, small intestine, pancreas, and kidney. Preferably, the organ or tissue is the blood-brain barrier (BBB).

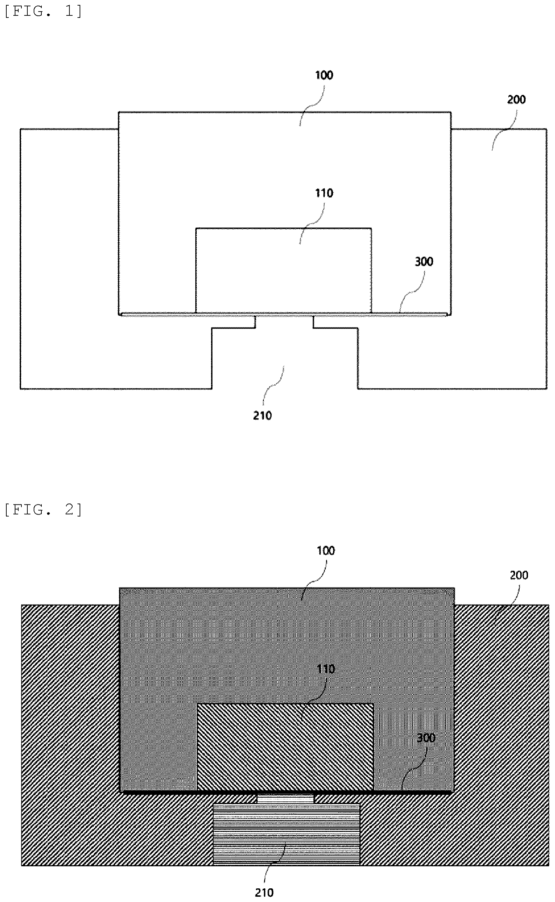

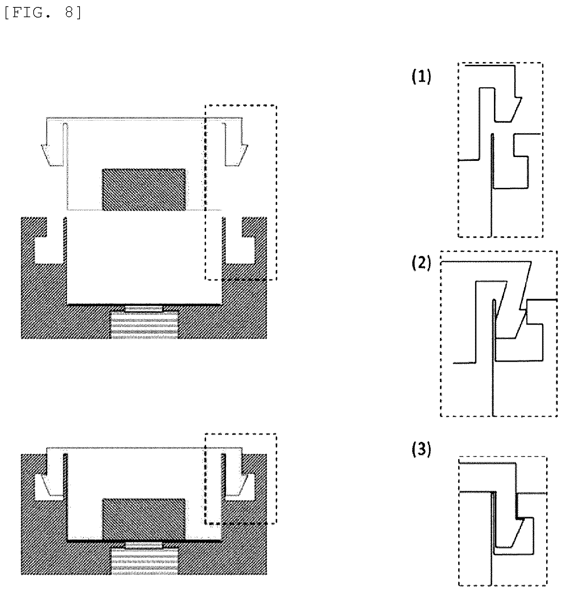

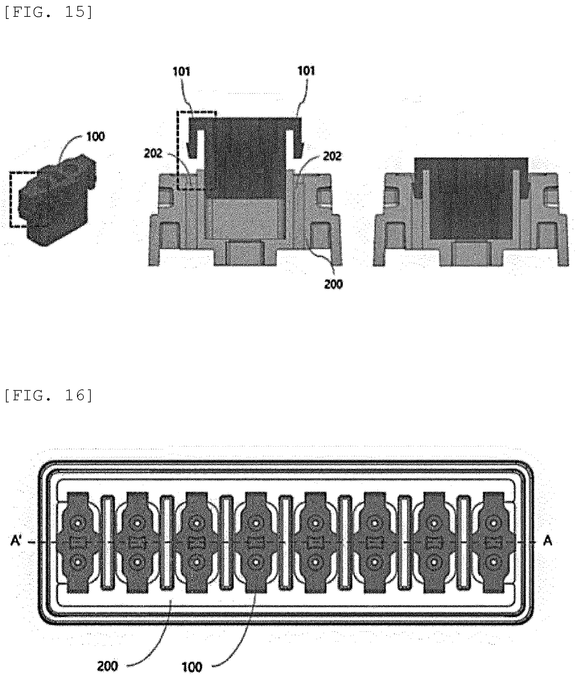

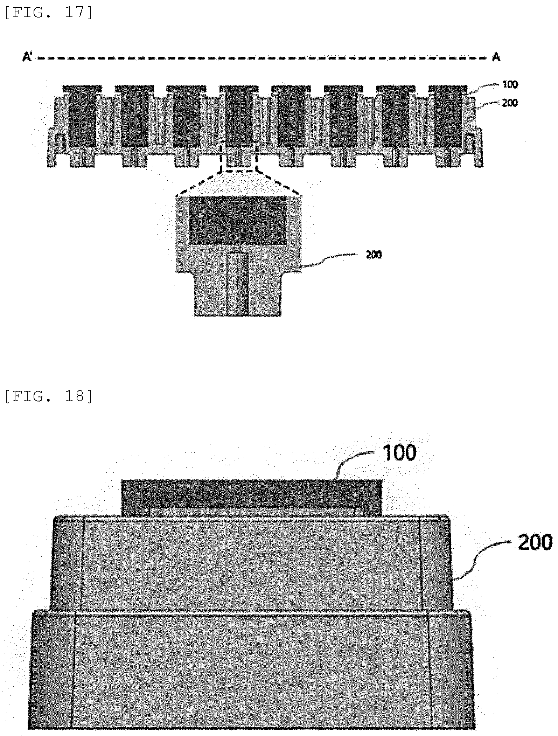

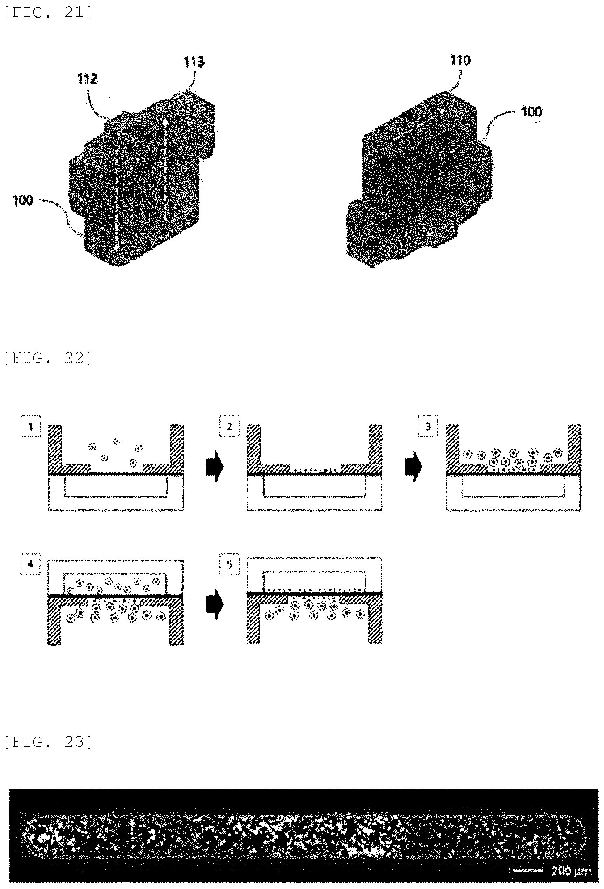

Hereinafter, the microfluidic device of the present invention will be described in detail with reference to the drawings. The present invention may include the microfluidic device embodied in FIGS. 1 to 21, but is not limited thereto. In particular, although specific embodiments of the microfluidic device of the present invention are shown in FIGS. 22 and 23, the present invention is not limited to these embodiments and encompasses various modifications that may be implemented by those skilled in the art.

The microfluidic device of the present invention includes an insert 100, a base 200, and a porous membrane 300 positioned between the insert 100 and the base 200.

Referring to FIGS. 1 to 3, the insert 100 includes a first central channel 110, and the base 200 includes a first open chamber 210.

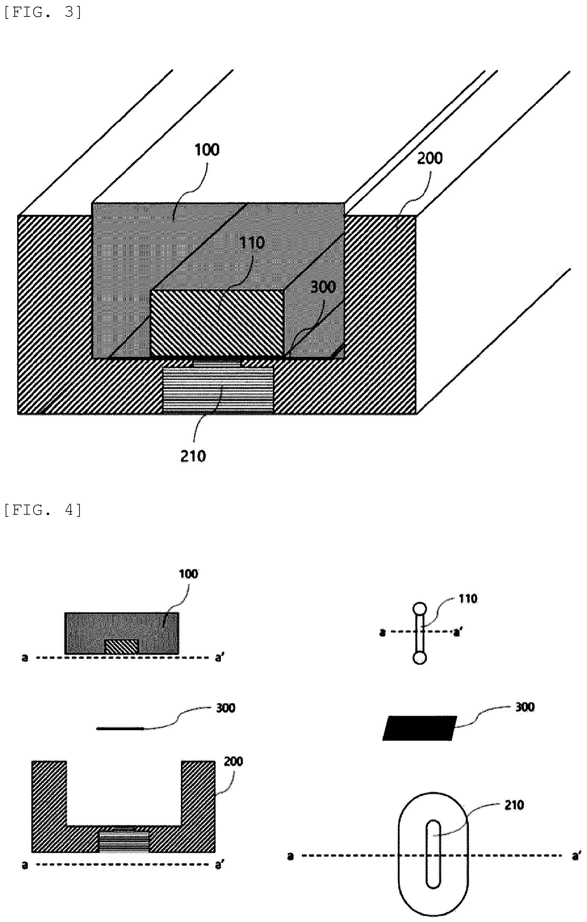

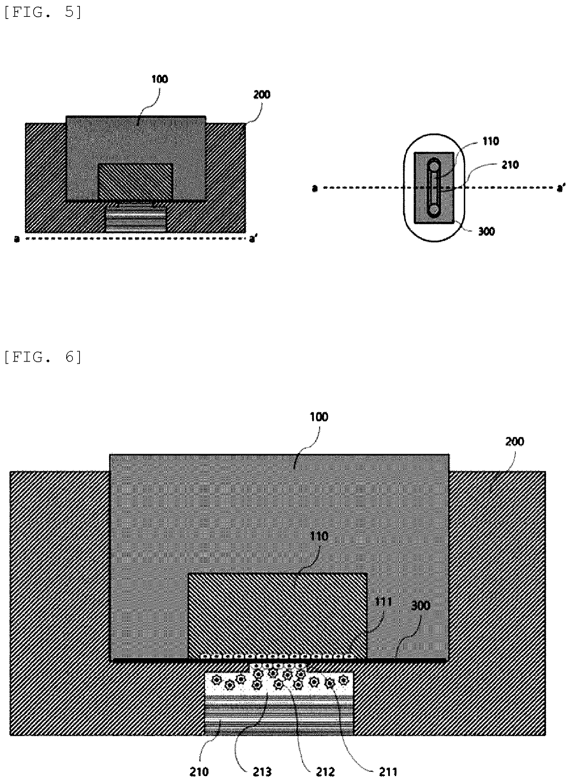

Referring to FIGS. 4 and 5, the porous membrane 300 of the microfluidic device may be disposed between the first central channel 110 and the first open chamber 210.

Referring to FIG. 6, the first central channel 110 of the microfluidic device may include first cells 111, and the first open chamber 210 may include second cells 211, third cells 212, and/or a hydrogel 213. In one embodiment, the first cells 111 may be attached to an upper surface of the porous membrane 300 and cultured, the second cells 211 may be attached to a lower surface of the porous membrane 300 and cultured, and the third cells 212 may be cultured in 3D within the hydrogel 213.

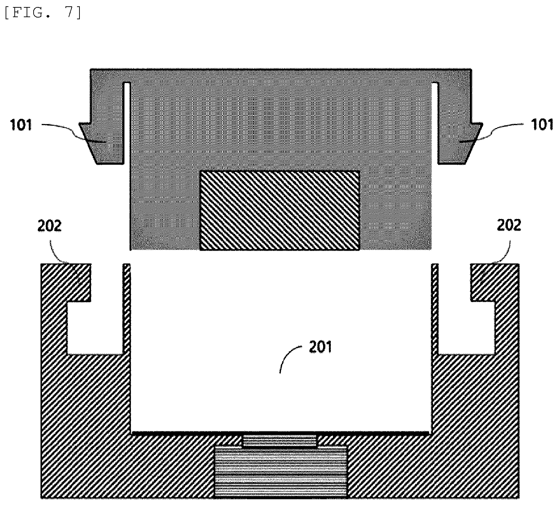

Referring to FIGS. 7 and 8 and FIG. 15, the base 200 of the microfluidic device may include an insertion groove 201 into which the insert 100 can be inserted, and the insert 100 may be detachable from the base 200. In addition, the insert 100 may include a first latching head 101, and the base 200 may include a second latching head 202, such that the first latching head 101 and the second latching head 202 may engage with each other, and the insert 100 and the base 200 may be detachably coupled in a snap-fit manner.

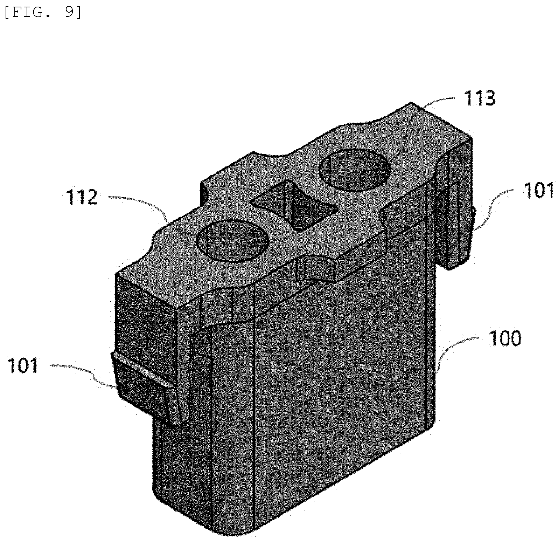

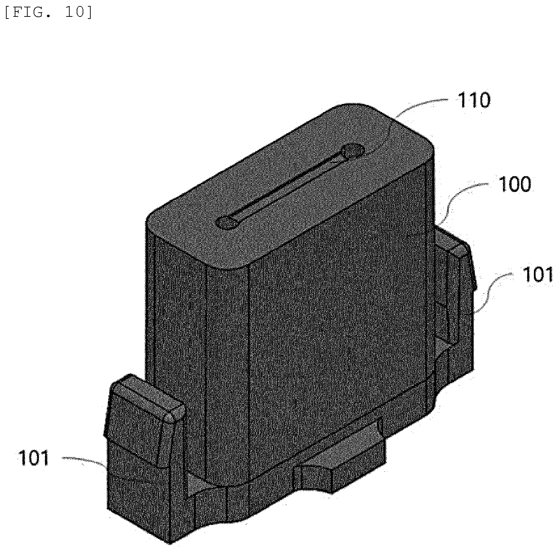

Referring to FIGS. 9 and 10, the insert 100 of the microfluidic device may include a first central channel inlet 112 and an outlet 113. The first central channel inlet 112 and the outlet 113 may be connected to the first central channel 110 in a tubular form.

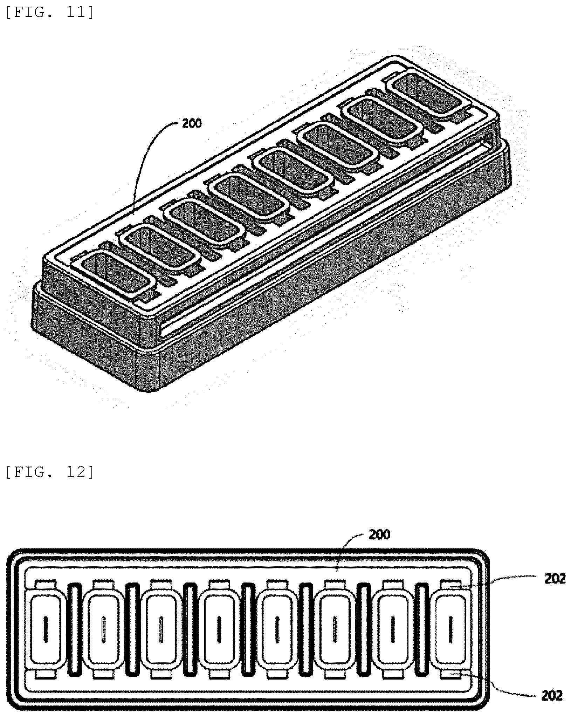

Referring to FIGS. 11 to 13 and FIGS. 15 to 19, the base 200 of the microfluidic device may have a structure in which one or more inserts 100 may be detachably coupled. Preferably, the base may be configured such that eight inserts 100 can be detachably coupled.

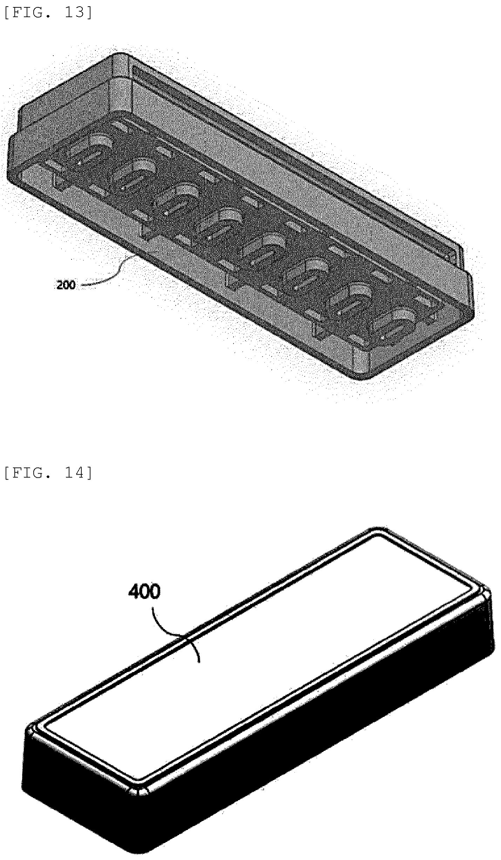

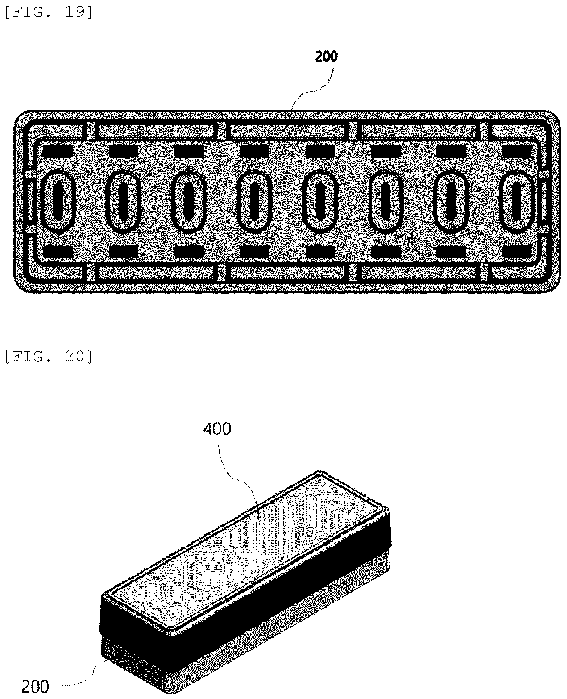

Referring to FIGS. 14 and 20, the microfluidic device may include a cover 400.

Referring to FIG. 21, a fluid may be injected through the first central channel inlet 112 included in the insert 100 of the microfluidic device, and the fluid may be perfused through the first central channel 110 toward the first central channel outlet 113.

The present invention will now be described in further detail through the following examples. However, these examples are provided for illustrative purposes only and are not intended to limit the scope of the present invention.

### (Example 1)

### Optimization of first open chamber structure design

To confirm the conditions under which liquid within the first open chamber would not leak out of the microfluidic device when the device was inverted, experiments were conducted to evaluate the width, length, depth, volume, and surface area in contact with air of the first open chamber.

The open chamber was fabricated by attaching a frame structure made of polydimethylsiloxane to a glass slide. A measured volume of water sufficient to fill the chamber was injected, and the chamber was inverted to determine whether the water was retained inside.

Under various conditions of width, length, depth, volume, and surface area of the open chamber, the retention of liquid after inversion was examined, and the results are presented in Table 1 below.

**[TABLE 1]**

| Open chamber design conditions | | | | | Retention of liquid when the chamber is inverted |
|---|---|---|---|---|---|
| Width (mm) | Length (mm) | Depth (mm) | Surface area (mm²) | Volume (µL) | |
| 4 | 9 | 4 | 36 | 144 | Retained |
| 4 | 9 | 5 | 36 | 180 | Retained |
| 4 | 9 | 6 | 36 | 216 | Not retained |
| 4 | 10 | 4 | 40 | 160 | Retained |
| 4 | 10 | 5 | 40 | 200 | Retained |
| 4 | 10 | 6 | 40 | 240 | Not retained |
| 4 | 11 | 4 | 44 | 175 | Retained |
| 4 | 11 | 5 | 44 | 220 | Retained |
| 4 | 11 | 6 | 44 | 264 | Not retained |
| 4 | 12 | 4 | 48 | 192 | Not retained |
| 4 | 12 | 5 | 48 | 240 | Not retained |
| 4 | 12 | 6 | 48 | 288 | Not retained |
| 6 | 6 | 5 | 36 | 180 | Retained |

As shown in Table 1, liquid was retained within the chamber when the volume was 220 µL or less and the surface area was 44 mm² or less.

When the chamber was completely filled with water and inverted, the liquid remained in the chamber when the surface tension acting on the liquid exceeded the gravitational force. Accordingly, it was confirmed that the volume of liquid retained increased with increasing surface tension acting on the liquid.

The present invention is intended to form a tissue barrier by culturing cells on both the upper and lower surfaces of a porous membrane within the microfluidic device. Therefore, during the step of attaching cells to the upper surface of the porous membrane, it is essential that the liquid in the lower open chamber be stably retained.

Accordingly, it was confirmed that the volume of the first open chamber of the microfluidic device is preferably 220 µL or less and the surface area is 44 mm² or less.

### (Example 2)

### Fabrication of blood-brain barrier (BBB)-mimetic microfluidic device

Using the assembled microfluidic device shown in FIGS. 1 to 21, a microfluidic device capable of mimicking the blood-brain barrier (BBB) was fabricated as follows.

The process of forming a blood-brain barrier-mimetic model using the microfluidic device is illustrated in FIG. 22. Specifically, (1) the microfluidic device was inverted, and 15 µL of human brain vascular pericytes (HBVPs) at a concentration of 1×10⁶ cells/mL were injected into the first open chamber. (2) The device was incubated for 30 minutes to ensure uniform attachment of the pericytes to the surface of the porous membrane, followed by replacement of the culture medium. (3) With culture medium present in the first open chamber, 4 µL of human astrocytes (HAs) embedded in Matrigel at a concentration of 5×10⁶ cells/mL were injected into the first open chamber and cultured for 30 minutes to allow the Matrigel to solidify. (4) The microfluidic device was inverted again, and 7 µL of induced pluripotent stem cell-derived human brain microvascular endothelial cells (iPSC-derived HBMECs) at a concentration of 7×10⁷ cells/mL were injected into the first central channel. (5) The device was cultured for 1 hour, after which the culture medium was replaced.

To confirm the formation of a blood-brain barrier-mimetic model through co-culture of human brain vascular pericytes, human astrocytes, and human cerebral microvascular endothelial cells derived from induced pluripotent stem cells in the microfluidic device, the co-culture of human astrocytes and human cerebral microvascular endothelial cells inside the microfluidic device was fluorescently visualized and examined using a confocal microscope (LSM 700, Carl Zeiss, Oberkochen, Germany).

Consequently, as shown in FIG. 23, when the microfluidic device was inverted and human brain microvascular endothelial cells derived from induced pluripotent stem cells were cultured in the first central channel, it was confirmed that astrocytes embedded in Matrigel and culture medium injected into the first open chamber were retained within the chamber and cultured, despite being positioned against gravity.

### [Description of Reference Numerals]

- 100:: Insert
- 101:: First latching head
- 110:: First central channel
- 111:: First cell
- 112:: First central channel inlet
- 113:: First central channel outlet
- 200:: Base
- 201:: Insertion groove
- 202:: Second latching head
- 210:: First open chamber
- 211:: Second cell
- 212:: Third cell
- 213:: Hydrogel
- 300:: Porous membrane
- 400:: Cover

## Claims

1. A microfluidic device comprising:
an insert including a first central channel;
a base including a first open chamber; and
a porous membrane positioned between the insert and the base.

2. The microfluidic device according to claim 1, wherein the insert is detachable from the base.

3. The microfluidic device according to claim 2, wherein the base comprises a groove into which the insert is inserted.

4. The microfluidic device according to claim 2, wherein the insert comprises a first latching head,
the base comprises a second latching head, and
wherein the first latching head and the second latching head engage with each other so that the insert and the base are detachably coupled in a snap-fit manner.

5. The microfluidic device according to claim 1, wherein the first central channel comprises an inlet and an outlet configured to induce and control perfusion of a liquid.

6. The microfluidic device according to claim 1, wherein the first central channel comprises tissue barrier cells.

7. The microfluidic device according to claim 6, wherein the tissue barrier cells are at least one selected from the group consisting of vascular endothelial cells, skin cells, cancer cells, glandular cells, muscle cells, and epithelial cells of the bronchial tubes, colon, small intestine, pancreas, and kidney.

8. The microfluidic device according to claim 1, wherein the first open chamber is open at an upper portion and in fluid communication with the outside.

9. The microfluidic device according to claim 1, wherein the first open chamber comprises internal tissue cells and hydrogels.

10. The microfluidic device according to claim 9, wherein the internal tissue cells are at least one selected from the group consisting of astrocytes, pericytes, neurons, neural stem cells, glial cells, cardiomyocytes, smooth muscle cells, intestinal epithelial cells, keratinocytes, dermal fibroblasts, podocytes, and glomerular endothelial cells.

11. The microfluidic device according to claim 9, wherein the hydrogel is at least one selected from the group consisting of collagen, laminin, hyaluronic acid, minerals, fibrin, fibronectin, elastin, peptides, polyethylene glycol, and alginate.

12. The microfluidic device according to claim 1, wherein the first open chamber has a volume of 220 µL or less.

13. The microfluidic device according to claim 1, wherein the first open chamber has a surface area of 44 mm² or less.

14. The microfluidic device according to claim 1, wherein the insert is separated from the base, and one or more cells selected from the group consisting of cells included in the first central channel, cells included in the first open chamber, and cells attached to the porous membrane are respectively isolated.

15. The microfluidic device according to claim 1, wherein the microfluidic device is made of plastic, glass, metal, or silicon.

16. A method for culturing cells in a microfluidic device, comprising:
assembling a microfluidic device comprising an insert including a first central channel, a base including a first open chamber, and a porous membrane positioned between the insert and the base, wherein the insert is detachable from the base;
inverting the microfluidic device, then injecting second cells into the first open chamber, and culturing the cells;
injecting a hydrogel including third cells into the first open chamber, and then culturing the cells; and
inverting the microfluidic device again, then injecting first cells into the first central channel, and culturing the cells.

17. The method according to claim 16, wherein:
the first central channel comprises an inlet and an outlet configured to induce and control perfusion of a liquid;
the first open chamber is open at an upper portion and in fluid communication with the outside;
the hydrogel including the second cells and the third cells is injected through the upper portion of the first open chamber, and
the first cells are injected through the first central channel inlet.

18. The method according to claim 16, wherein:
the first cells are selected from the group consisting of vascular endothelial cells, skin cells, cancer cells, glandular cells, muscle cells, and epithelial cells of the bronchial tube, colon, small intestine, pancreas, or kidney;
the second cells are selected from the group consisting of pericytes, glial cells, cardiomyocytes, smooth muscle cells, intestinal epithelial cells, keratinocytes, dermal fibroblasts, podocytes, and glomerular endothelial cells; and
the third cells are selected from the group consisting of astrocytes, neurons, and neural stem cells.

19. The method according to claim 16, wherein the first open chamber has a volume of 220 µL or less.

20. The method according to claim 16, wherein the first open chamber has a surface area of 44 mm² or less.

21. A method for mimicking an organ or a tissue in a microfluidic device which comprises: an insert including a first central channel; a base including a first open chamber; and a porous membrane positioned between the insert and the base,
wherein the insert is detachable from the base.

22. The method according to claim 21, wherein the first open chamber has a volume of 220 µL or less and a surface area of 44 mm² or less.
